(19) Europäisches Patentamt
European Patent Office
Office européen des brevets

(11) Publication number: **0 342 984 A2**

(12) **EUROPEAN PATENT APPLICATION**

(21) Application number: **89305054.2**

(22) Date of filing: **18.05.89**

(51) Int. Cl.4: **C 12 Q 1/32**
C 12 Q 1/00

(30) Priority: **18.05.88 JP 121458/88**

(43) Date of publication of application:
**23.11.89 Bulletin 89/47**

(84) Designated Contracting States:
**AT BE CH DE ES FR GB GR IT LI LU NL SE**

(71) Applicant: **Kyowa Medex Co. Ltd.**
**6-1 Ohtemachi Itchome Chiyoda-ku**
**Tokyo (JP)**

(72) Inventor: **Aoyama, Norihito**
**410-1, Nameri, Nagaizumi-cho**
**Sunto-gun Shizuoka-ken (JP)**

**Tatano, Toshio**
**2297-6, Ooka**
**Numazu-shi Shizuoka-ken (JP)**

**Miike, Akira**
**410-1, Nameri, Nagaizumicho**
**Sunto-gun Shizuoka-ken (JP)**

(74) Representative: **Lambert, Hugh Richmond et al**
**D. YOUNG & CO. 10 Staple Inn**
**London, WC1V 7RD (GB)**

(54) **Method for the determination of NAD(P)H.**

(57) The invention provides a method and kit for the determination of NAD(P)H in a sample, e.g. a clinical sample, which comprises reducing pyruvic acid or a salt thereof with NAD(P)H in the presence of lactate dehydrogenase to form lactic acid, oxidizing the lactic acid in the presence of lactate oxidase to form hydrogen peroxide and determining the hydrogen peroxide by reaction with peroxidase in the presence of a chromogen unsusceptible to NAD(P)H. The method is especially useful in determination of analytes, e.g. bile acid, phosphohexose isomerase, in serum containing lactic acid, in which case the sample is initially reacted with lactate oxidase to convert the lactic acid to pyruvic acid and $H_2O_2$, which is then decomposed, the pyruvic acid produced in that case providing at least a proportion of the pyruvic acid which is subsequently converted back to lactic acid.

FIG.1

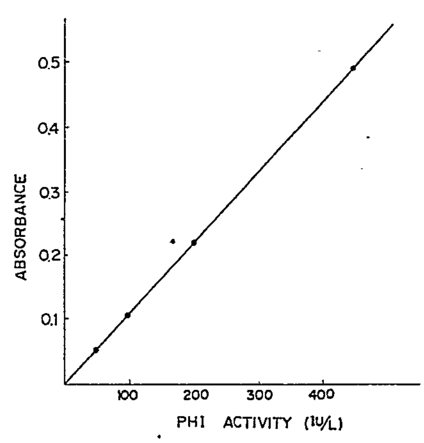


EP 0 342 984 A2

## Description

### METHOD FOR THE DETERMINATION OF NAD(P)H

The present invention relates to a method for the determination of the reduced form of nicotinamide adenine dinucleotide (phosphate) [hereinafter referred to as NAD(P)H] in a sample, e.g. a clinical sample.

The present invention is particularly applicable to the determination of substrate or enzyme activity in a sample via an enzyme reaction in which NAD(P)H is stoichiometrically produced or consumed, for example, the determination of either phosphohexose isomerase or total bile acid in serum samples.

With regard to the determination of NAD(P)H, the following methods are known: (1) directly measuring absorption of NAD(P)H in the ultraviolet region; (2) linking the reaction of NAD(P)H via an electron transfer mechanism to a flourescent substance and measuring the fluorescence; (3) linking the reaction of NAD(P)H via an electron transfer to a tetrazolium salt and colorimetrically determining the formazan produced; (4) reacting NAD(P)H with an electron transfer reaction of NAD(P)H in the presence of superoxide dismutase and determining hydrogen peroxide formed: [Rinsho Kagaku, 15 (1), 20-27 (1986), JP-A-210899/1984 and 19100/1987]; (5) allowing a leuco type compound to develop colour in the presence of peroxidase and diaphorase and colorimetrically determining the colour (JP-A-80600/1985); (6) reducing a pyridine enzyme substrate with pyridine enzyme, reacting the reduced substrate with an oxidase to form hydrogen peroxide and determining the hydrogen peroxide (JP-A-138200/1987); and (7) determining hydrogen peroxide formed by use of glutamate dehydrogenase and glutamate oxidase (JP-A-43398/1985).

These methods suffer from the following defects: sensitivity is poor; the determination systems are susceptible to other components in a sample; and the application to continuous assays is difficult. A particular difficulty arises in the case of samples, e. g. clinical samples such as serum and which may contain lactic acid in large quantities, and also possibly lactate dehydrogenase. In those cases the lactic acid undergoes reaction with lactate dehydrogenase in the presence of NAD(P) to produce NAD(P)H which is indistinguishable from the NAD(P)H which is to be determined. In those cases a blank sample test is necessary.

The present invention provides a method for determination of NAD(P)H in a sample containing lactic acid and which requires no blank sample test. Broadly speaking this method comprises initially reacting lactic acid in the sample with lactate oxidase (hereinafter referred to as LOX) to form pyruvic acid and hydrogen peroxide. That hydrogen peroxide is decomposed using a hydrogen peroxide decomposer, following which the pyruvic acid, as such or in salt form, is reacted in situ, i.e. in the NAD(P)H containing sample, with lactate dehydrogenase (hereinafter referred to as LDH) to reform lactic acid in an amount which is directly proportional to the NAD(P)H content of the sample. That lactic acid is then oxidised in the presence of LOX to form hydrogen peroxide which can then be determined by reacting with peroxidase in the presence of a chromogen unsusceptible to NAD(P)H.

The reactions involved in the method are illustrated by the following reaction equations.

$$\text{lactic acid (in a sample)} + O_2 \xrightarrow{\text{LOX}} \text{pyruvic acid} + \underset{\text{decomposed}}{H_2O_2}$$

$$\text{pyruvic acid} + \text{NAD(P)H} \xrightarrow{\text{LDH}} \text{lactic acid} + \text{NAD(P)}$$

$$\text{lactic acid} + O_2 \xrightarrow{\text{LOX}} \text{pyruvic acid} + \underset{\text{determined}}{H_2O_2}$$

Whilst as above described the method of the invention has been devised specifically with reference to the determination of NAD(P)H in samples, e.g. clinical samples, containing lactic acid, and in which that lactic acid is initially converted to pyruvic acid, it will be apparent that the method can be applied to samples free from lactic acid, and in which case the invention resides broadly in a method for the determination of NAD(P)H in a sample, which comprises adding pyruvic acid in free acid or salt form to the sample, if not already present or produced in situ therein, reacting the pyruvic acid in the sample with lactate dehydrogenase in the presence of said NAD(P)H thereby to convert the pyruvic acid quantitatively into lactic acid, and the NAD(P)H quantitatively into NAD(P), reacting the lactic acid produced with lactate oxidase thereby quantitatively forming hydrogen peroxide, and determining the hydrogen peroxide produced by the reaction thereof with a peroxidase in the presence of a chromogen unsusceptible to NAD(P)H.

It will also be apparent that, in the case where lactic acid is initially present in the sample, it may be necessary, in the second step, to add additional pyruvic acid to the sample, but this will depend upon the relative proportions of lactic acid and NAD(P)H initially present in the sample, and consequently the quantity of

pyruvic acid that is produced in situ by the initial oxidation of the lactic acid by the LOX.

The method of the present invention is particularly applicable to the determination of bile acid or phosphohexose isomerase in serum samples, and in which case the method comprises:

(a) oxidising lactic acid in the sample in the presence of lactate oxidase into pyruvic acid to form hydrogen peroxide;

(b) decomposing the hydrogen peroxide;

(c) subjecting the bile acid or phosphohexose isomerase, as the case may be, to an enzyme reaction with the appropriate enzyme or substrate, respectively, In the presence of NAD(P) stoichiometrically to form NAD(P)H;

(d) reducing pyruvic acid or a salt thereof with the NAD(P)H so formed in the presence of lactate dehydrogenase to form lactic acid;

(e) oxidising the lactic acid in the presence of lactate oxidase to form hydrogen peroxide; and

(f) colorimetrically determining the hydrogen peroxide by reaction with peroxidase in the presence of a chromogen unsusceptible to NAD(P)H.

In a further aspect of the present invention there is provided a reagent kit for the determination of NAD(P)H in a sample, the reagent kit comprising as a first reagent: lactate oxidase, an $H_2O_2$ decomposer, and one or other of peroxidase and a chromogen unsusceptible to NAD(P)H, and as a second reagent: the other of said peroxidase and chromogen, whichever is not included in the first component, the first or second reagents or both containing one or other or both of lactic acid dehydrogenase and pyruvic acid or a salt thereof, with the proviso that the two reagents between them contain both said lactic acid dehydrogenase and said pyruvic acid or salt.

Optionally, the reagent kit may also contain as components of the second reagent NAD(P), an enzyme or substrate capable of reacting respectively with an enzyme substrate or enzyme contained within a sample and in the presence of said NAD(P), thereby to convert the NAD(P) component of the second reagent when added to the sample to NAD(P)H.

In the method of the invention, the reaction of LOX proceeds much more rapidly than that of LDH, so that NAD(P)H can also be accurately determined, even if LDH and pyruvic acid or a salt thereof are incorporated in the first reagent.

In case that NAD(P)H to be determined is formed through other enzyme reactions, the enzyme, substrate, and other cofactors necessary for the enzyme reaction can be added to the sample together with the reagents for determining the NAD(P)H. For example, a first reagent comprising LOX, $H_2O_2$ decomposer, LDH, pyruvic acid or a salt thereof and peroxidase, and a second reagent comprising that component, i.e. enzyme or substrate, depending on whether the analyte to be determined is an enzyme or enzyme substrate, necessary for the enzyme reaction, and a chromogen unsusceptible to NAD(P)H can be added to the sample. The $H_2O_2$ produced can be determined in the same way as described above. Among the components necessary for the enzyme reaction of the analyte to form NAD(P)H, components that do not interfere in the lactic acid-decomposing reaction in a sample and are not otherwise detrimental to the determination, may be incorporated into the first reagent.

As the $H_2O_2$-decomposer, catalase is preferred although other compounds are usable so long as they do not interfere in the enzyme reactions. As examples of such compounds, mention may be made of phenol derivatives, aniline derivatives and naphthol derivatives, which are reacted with $H_2O_2$ in the presence of peroxidase without forming any pigment. In particular, it is convenient to use, as $H_2O_2$ decomposer, one constituent of a coupling agent which is reacted with $H_2O_2$ in the presence of peroxidase to form a pigment, because when one constituent of the coupling agent is added, $H_2O_2$ is decomposed but no pigment is formed until the other constituent of the coupling agent is added. Thus $H_2O_2$ derived from the analyte can be determined by adding the other constituent of the coupling agent as a chromogen.

Following decomposition of the $H_2O_2$ in the first step, it may or may not be necessary to add a deactivator to deactivate the $H_2O_2$ decomposer. In the case of the preferred $H_2O_2$ decomposer, catalase, sodium azide is the preferred deactivator.

The present invention is generally carried out in a 0.005 to 2 mol/l buffer having a pH of 2 to 11, such as phosphate, Tris-hydrochloride, succinate, Bis(2-hydroxyethyl)iminotris-(hydroxymethyl)methane [Bis-Tris], N-2-Hydroxyethylpiperazine-N′-2-ethanesulfonic acid [HETES], acetate, Good, etc., at around the optimum temperature of enzyme, generally at 5 to 50°C, preferably at 25 to 40°C.

Concentrations of the respective constituents in the reagent solutions are shown:

| | |
|---|---|
| LDH: | 0.1 - 100 IU/ml |
| LOX: | 0.1 - 1000 IU/ml |
| Pyruvic acid or a salt thereof: | 0.1 100 mM/l |
| Peroxidase: | 0.1 - 1000 IU/ml |
| Chromogen: | more than equimolar amount of NAD(P)H to be determined, preferably 10 to 1000 molar equivalents |

When an acidic buffer is used in the present invention, the reaction proceeds toward:

$$\text{pyruvic acid} + \text{NAD(P)H} \xrightarrow{\text{LDH}} \text{lactic acid} + \text{NAD(P)}$$

and hence, NAD(P)H is not produced from lactic acid and NAD(P) in a sample. Even though other NAD(P)H-producing reaction systems, involving other components, may be present in the sample in addition to the reaction system of the present invention and the NAD(P)H-producing reaction proceeds, then when the first reagent containing LDH, pyruvic acid or salt thereof, LOX and $H_2O_2$-decomposer is added, the produced NAD(P)H and pyruvic acid are changed to NAD(P) and lactic acid, respectively by the action of LDH and the lactic acid is decomposed by LOX. The formed $H_2O_2$ is decomposed by the $H_2O_2$-decomposer, and the second reagent containing components necessary for an enzyme reaction of the analyte is added.

Reducing substances, for example bilirubin, glutathione, etc. which may be present in the sample, are also decomposed by $H_2O_2$ formed by addition of the first reagent. Thus, there is no adverse effect on the determination of NAD(P)H derived from the analyte. The method of the invention is therefore extremely tolerant of other components which may be present in the sample.

The absorbance of the reaction solution after reaction with the chromogen in the presence of peroxidase is measured at the maximum absorption wavelength of the formed pigment, and in contrast to using reagent blanks as a control, the NAD(P)H content of the sample can be determined from a calibration curve prepared from test samples containing known concentrations of NAD (P)H.

In general, NAD(P)H in the reaction solution will preferably be adjusted with a reagent solution or distilled water to a concentration of 0.00001 to 1 mg/ml.

In the reaction system, a surfactant such as polyethylene glycol-mono-p-isooctyl phenyl ether (trademark, Triton ×-100), etc. may optionally be used.

A small quantity of phenol may be added to the reaction system for the purpose of activating peroxidase or accelerating the pigment-forming.

In addition, activators for the enzymes used in the NAD(P)H-producing reaction, such as magnesium chloride and/or stabilizers for the enzyme, such as albumin, are used in 0.01 to 20 mg/ml.

In the present invention, chromogens which are unsusceptible to NAD(P)H are used for determination of $H_2O_2$ in the presence of peroxidase. Further, due to a sample from living body, chromogens having the maximum absorption wavelength distant from the maximum absorption wavelength of hemoglobin, bilirubin, etc., especially those having max of 600 to 750 nm are preferred. As such chromogens, mention can be made of the compounds described in JP-A-29297/82 (US Patent No. 4,384,042, EP-B-0 045 220) and JP-A-1 82361/84 (US Patent Application Serial No. 065,126, EP-A-0,124,287) and compounds represented by general formula (1):

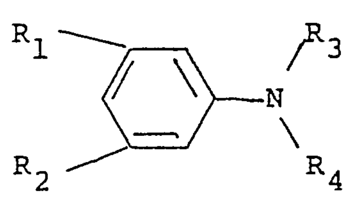

(I)

wherein $R_1$ and $R_2$ independently represent hydrogen or alkyl having 1 to 6 carbon atoms, for example, methyl, ethyl, propyl, butyl, etc., and $R_3$ and $R_4$ independently represent sulfoalkyl wherein the alkyl moiety has the same significance as in $R_1$.

Representative examples of the chromogen which can be used in the present invention and $\lambda$max thereof are shown in Tables 1, 2, and 3.

Compounds shown in Table 1 are those described in JP-A-29297/82 (U.S. Patent No. 4,384,042, EP-B-0045220); symbols appearing in the table have the following definitions.

I : 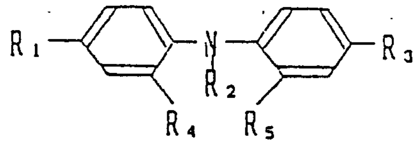

II : 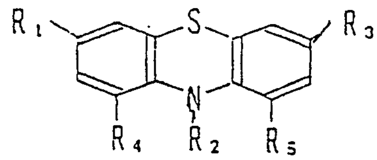

A : $N(CH_3)_2$

B : $N(C_2H_5)_2$

D : 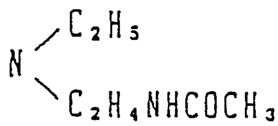

$Y_p$ : 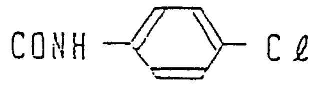

E : 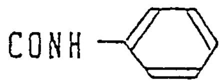

$Y_m$ : 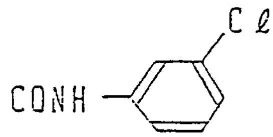

F : 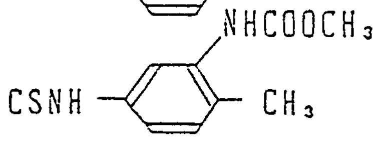

$Y_o$ : 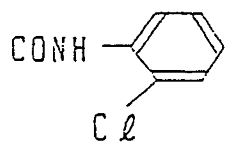

G : 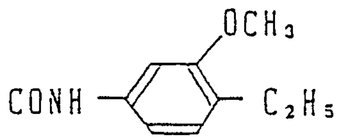

$Y_B$ : 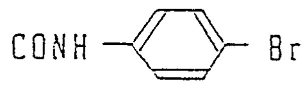

K : 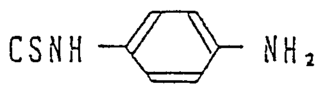

$Y_s$ : 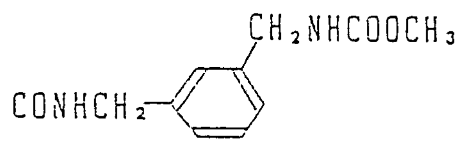

L : 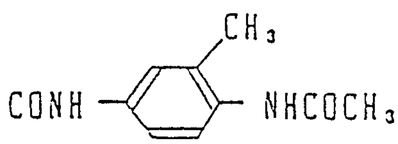

M : 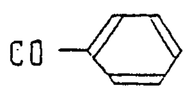

P : 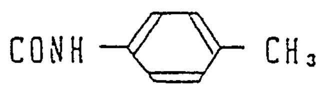

Q : 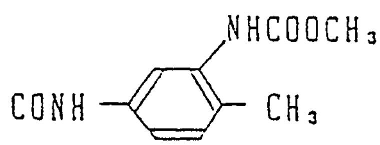

T : $CONHCH_3$

Z : $CONHCH_2COOH$

Table 1

| Compound No. | Formula | $R_1$ | $R_2$ | $R_3$ | $R_4$ | $R_5$ | λmax |
|---|---|---|---|---|---|---|---|
| 1 | II | A | $Y_s$ | A | H | H | 665 |
| 2 | I | $NH_2$ | H | A | H | H | 700 |
| 3 | I | A | H | A | $CH_3$ | $CH_3$ | 720 |
| 4 | I | A | E | B | H | H | 720 |
| 5 | I | D | F | D | H | H | 730 |
| 6 | I | D | H | D | $CH_3$ | $CH_3$ | 730 |
| 7 | I | A | G | OH | $CH_3$ | H | 600 |
| 8 | I | OH | K | OH | $CH_3$ | H | 600 |
| 9 | I | A | L | B | H | OH | 720 |
| 10 | II | A | H | A | H | H | 665 |
| 11 | II | A | M | A | H | H | 665 |
| 12 | II | B | P | D | $CH_3$ | H | 670 |
| 13 | II | D | Q | D | H | H | 670 |
| 14 | II | D | Q | OH | $CH_3$ | $CH_3$ | 620 |
| 15 | II | A | $Y_P$ | A | H | H | 665 |
| 16 | II | A | $Y_O$ | A | H | H | 665 |
| 17 | II | A | $Y_m$ | A | H | H | 665 |
| 18 | II | A | $Y_B$ | A | H | H | 665 |
| 19 | II | A | E | A | H | H | 665 |
| 20 | II | A | T | A | H | H | 665 |
| 21 | II | A | Z | A | H | H | 665 |

Compounds shown in Table 2 are those described in JP-A-182361/84 (U.S. Patent Application Serial No. 065,126, EP-A-0124287); symbols appearing in the table have the following definitions.

I : 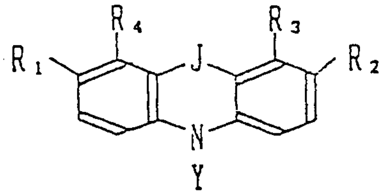

II : 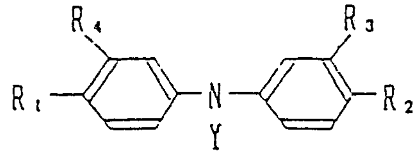

M : $CH_3$

Q : 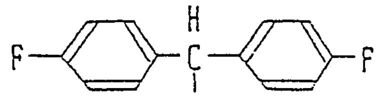

E : $C_2H_5$

T : 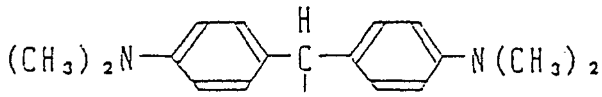

B Z : 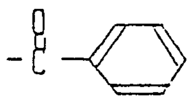

U : 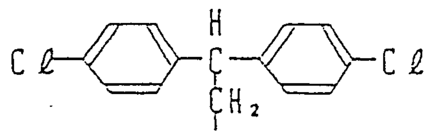

V : 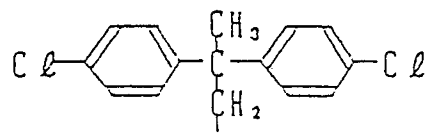

D : $CH_2CH_2CH_2SO_3H$

W : 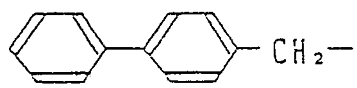

X : 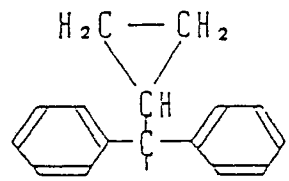

A : 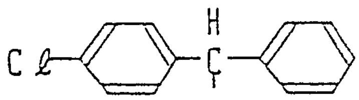

F : 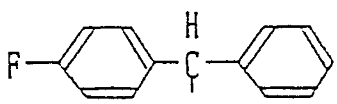

Z : 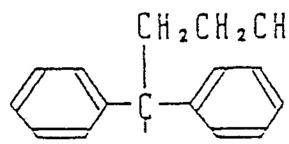

K : 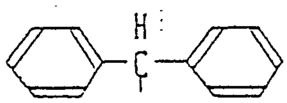

α : 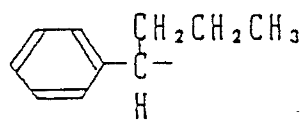

L : 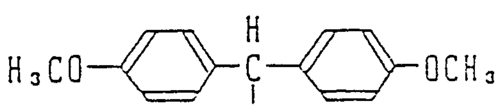

β : 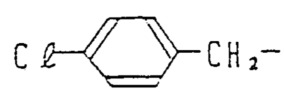

P : 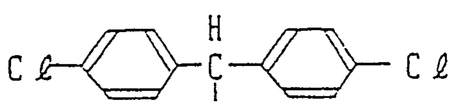

γ : 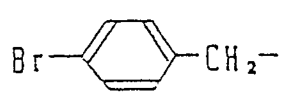

Table 2

| Compound NO. | Formula | J | $R_1$ | $R_2$ | $R_3$ | $R_4$ | Y | λmax |
|---|---|---|---|---|---|---|---|---|
| 22 | II | – | $NM_2$ | $NM_2$ | A | A | H | 747 |
| 23 | II | – | $NM_2$ | $NM_2$ | F | F | H | 740 |
| 24 | II | – | $NM_2$ | $NM_2$ | K | K | H | 735 |
| 25 | II | – | $NM_2$ | $NM_2$ | L | L | H | 700 |
| 26 | II | – | $NM_2$ | $NM_2$ | P | P | H | 755 |
| 27 | II | – | $NM_2$ | $NM_2$ | Q | Q | H | 745 |
| 28 | II | – | $NM_2$ | $NM_2$ | T | T | H | 520 |
| 29 | II | – | $NM_2$ | $NM_2$ | P | H | H | 750 |
| 30 | II | – | $NH_2$ | $NH_2$ | K | K | H | 720 |
| 31 | II | – | $NM_2$ | $NM_2$ | U | H | H | 728 |
| 32 | II | – | $NM_2$ | $NM_2$ | V | H | H | 690 |
| 33 | II | – | $NM_2$ | $NM_2$ | W | W | H | 733 |
| 34 | II | – | $NM_2$ | $NM_2$ | X | H | H | 728 |
| 35 | II | – | $NM_2$ | $NM_2$ | Z | H | H | 725 |
| 36 | II | – | $NM_2$ | $NM_2$ | α | H | H | 747 |
| 37 | II | – | $NM_2$ | $NM_2$ | β | β | H | 737 |
| 38 | II | – | $NM_2$ | $NM_2$ | γ | γ | H | 735 |
| 39 | I | S | $NM_2$ | $NM_2$ | P | H | H | 680 |
| 40 | I | S | $NM_2$ | $NM_2$ | P | $NO_2$ | H | 670 |
| 41 | II | – | $NH_2$ | OH | P | H | H | 700 |
| 42 | II | – | $NH_2$ | $OCH_3$ | P | H | H | 690 |
| 43 | II | – | $NH_2$ | $OCH_3$ | Q | H | H | 723 |
| 44 | II | – | $NH_2$ | OH | Q | H | H | 715 |
| 45 | II | – | $NH_2$ | $NH_2$ | P | P | H | 708 |
| 46 | II | – | $NH_2$ | $NH_2$ | Q | Q | H | 700 |
| 47 | II | – | $NH_2$ | $NH_2$ | A | A | H | 710 |
| 48 | II | – | $NH_2$ | $NH_2$ | F | F | H | 715 |
| 49 | II | – | $NM_2$ | $NM_2$ | P | P | BZ | 755 |
| 50 | I | S | $NM_2$ | $NM_2$ | P | H | BZ | 680 |
| 51 | I | O | $NE_2$ | $NE_2$ | P | H | BZ | 685 |

| | | | | | | | | |
|---|---|---|---|---|---|---|---|---|
| 52 | II | - | $ND_2$ | $ND_2$ | K | K | H | 760 |
| 53 | II | - | $ND_2$ | $ND_2$ | P | P | H | 770 |
| 54 | II | - | NHD | NHD | K | K | H | 710 |
| 55 | II | - | NHD | NHD | P | P | H | 720 |

Symbols appearing in Table 3 have the following definitions in general formula (1) and the compounds indicated in Table 3 are used in combination with 4-amino-antipyrine.

General formula (I)

A: $-CH_2CH_2CH_2SO_3H$

B: $-CH_2CHCH_2SO_3H$

OH

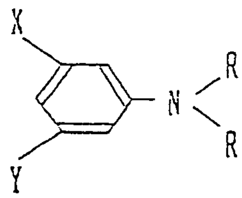

Table 3

| Compound No. | X | Y | R | λmax |
|---|---|---|---|---|
| 56 | H | H | A | 560 |
| 57 | H | $CH_3$ | A | 555 |
| 58 | H | $OCH_3$ | A | 545 |
| 59 | $CH_3$ | $CH_3$ | A | 633 |
| 60 | $OCH_3$ | $OCH_3$ | A | 595 |
| 61 | H | H | B | 565 |
| 62 | H | $CH_3$ | B | 556 |
| 63 | H | $OCH_3$ | B | 547 |
| 64 | $CH_3$ | $CH_3$ | B | 636 |
| 65 | $OCH_3$ | $OCH_3$ | B | 598 |

Compound Nos. 56 to 65 can be synthesized by reacting Starting compound 1 with 2 to 10 molar equivalents of Starting compound 2 under the following condition.

Table 4

| No. | Starting compound 1 | Starting compound 2 | Reaction condition |
|---|---|---|---|
| 56 | aniline | propane-sultone | in acetonitrile, 50°C 3 days |
| 57 | m-toluidine | " | " |
| 58 | m-anisidine | " | " |
| 59 | 3,5-dimethylaniline | " | " |
| 60 | 3,5-dimethoxyaniline | " | " |
| 61 | aniline | sec-chlorohydrinsulphonic acid | in toluene, 80°C 1 day |
| 62 | m-toluidine | " | " |
| 63 | m-anisidine | " | " |
| 64 | 3,5-dimethylaniline | " | " |
| 65 | 3,5-dimethoxylaniline | " | " |

As mentioned above, the method of the present invention is applicable to the determination of any analyte involved in an enzyme reaction system which stoichiometrically produces NAD(P)H through the reaction. Many such reaction systems are known. As particular analytes to be determined there may be mentioned, for example, glucose, galactose, alcohols, malic acid, aldehyde, xanthine, cholesterol, bile acid, phosphohexose isomerase (PHI) and neutral lipid. Such reaction systems are illustratively shown below:

1. Glucose

$$\text{glucose} + \text{ATP} \xrightarrow{\text{hexokinase}} \text{glucose-6-phosphate} + \text{ADP}$$

$$\text{glucose-6-phosphate} + NAD^{\oplus} \xrightarrow{\text{glucose-6-phosphate dehydrogenase}} \text{D-glucono-}\delta\text{-lactone-6-phosphate} + NADH + H^{\oplus}$$

2. Galactose

$$\text{galactose} + NAD^{\oplus} \xrightarrow{\text{galactose dehydrogenase}} \text{D-galactono-}\gamma\text{-lactone} + NADH + H^{\oplus}$$

3. Alcohol

$$\underset{\text{(ethanol)}}{\text{alcohol}} + NAD^{\oplus} \xrightarrow{\text{alcohol dehydrogenase}} \underset{\text{(acetaldehyde)}}{\text{aldehyde}} + NADH + H^{\oplus}$$

4. Malic acid

$$\text{malic acid} + NAD^{\oplus} \xrightarrow{\text{malate dehydrogenase}} \text{oxaloacetic acid} + NADH + H^{\oplus}$$

5. Aldehyde

$$R\text{-}CHO + NAD^{\oplus} + H_2O \xrightarrow{\text{aldehyde dehydrogenase}} R\text{-}COO^{-} + NADH + 2H^{\oplus}$$

6. Xanthine

$$\text{xanthine} + NAD^{\oplus} + H_2O \xrightarrow{\text{xanthine dehydrogenase}} \text{uric acid} + NADH + H^{\oplus}$$

7. Neutral lipid and glycerol

$$\text{neutral lipid} + 3H_2O \xrightarrow{\text{lipoprotein lipase}} \text{glycerol} + \text{fatty acid}$$

$$\text{glycerol} + NAD^{\oplus} \xrightarrow{\text{glycerol dehydrogenase}} \text{dihydroxyacetone} + NADH + H^{\oplus}$$

8. Bile acid

$$3\alpha\text{-hydroxybile acid} + NAD^{\oplus} \xrightarrow{3\alpha\text{-hydroxysteroid dehydrogenase } (3\alpha\text{-HSD})} \text{3-ketobilic acid} + NADH + H^{\oplus}$$

9. Cholesterol

$$\text{free cholesterol} + NAD^{\oplus} \xrightarrow{\text{cholesterol dehydrogenase}} \text{cholestenone} + NADH + H^{\oplus}$$

10. PHI activity

$$\text{fructose-6-phosphate} \xrightarrow{\text{PHI}} \text{glucose-6-phosphate}$$

$$\text{glucose-6-phosphate} + NAD^{\oplus} \xrightarrow{\text{glucose-6-phosphate dehydrogenase}} \text{fructose-6-phosphate} + NADH + H^{\oplus}$$

Generally in those cases where the analyte to be determined is a substrate, the total quantity of $H_2O_2$ formed or a forming rate of $H_2O_2$ is determined; where the analyte is an enzyme, i.e. an enzyme activity level, the forming rate of $H_2O_2$ is measured.

Certain specific embodiments of the present invention are illustrated in the following representative examples.

Reference Example 1 (Determination of NADH)

In 100 ml of 50 ml Good's buffer (pH 6.25) were dissolved 1000 IU of peroxidase, 800 IU of lactate dehydrogenase, 500 IU of lactate oxidase, 50 mg of sodium pyruvate, 500 mg of Triton X-100, 5 mg of phenol and (1) 10 mg of Compound No. 1, (2) 10 mg of Compound No. 20, (3) 10 mg of Compound No. 21, (4) 10 mg of Compound No. 22, (5) 10 mg of Compound No. 24, (6) 10 mg of Compound No. 26, (7) 10 mg of Compound No. 45, (8) 10 mg of Compound No. 53, (9) 10 mg each of 4-aminoantipyrine and Compound No. 56, (10) 10 mg each of 4-aminoantipyrine and Compound No. 57, (11) 10 mg each of 4-aminoantipyrine and Compound No. 60 or (12) 10 mg each of 4-aminoantipyrine and Compound No. 65, to prepare a reagent solution.

Separately, 50 ml of 100, 200, 300, 500 or 1000 μmol/l NADH solution was prepared, and 3 ml each of the reagent solutions described above was added thereto. The mixture was allowed to stand at 37°C for 5 minutes, and absorbance of the reaction solution at λmax of the chromogen was measured in contrast to a reagent blank test as a control.

The results are shown in Table 5, wherein the figures denote absorbance.

Table 5

| | Concentration of NADH (µmol/l) | | | | | |
|---|---|---|---|---|---|---|
| | λmax (nm) | 100 | 200 | 300 | 500 | 1000 |
| 1 | 665 | 0.164 | 0.327 | 0.492 | 0.822 | 1.635 |
| 2 | 665 | 0.165 | 0.329 | 0.495 | 0.823 | 1.651 |
| 3 | 665 | 0.162 | 0.325 | 0.486 | 0.813 | 1.622 |
| 4 | 747 | 0.103 | 0.206 | 0.307 | 0.513 | 1.033 |
| 5 | 735 | 0.104 | 0.207 | 0.309 | 0.518 | 1.038 |
| 6 | 755 | 0.107 | 0.215 | 0.321 | 0.535 | 1.071 |
| 7 | 708 | 0.071 | 0.140 | 0.211 | 0.352 | 0.698 |
| 8 | 770 | 0.095 | 0.189 | 0.285 | 0.474 | 0.950 |
| 9 | 560 | 0.060 | 0.119 | 0.181 | 0.300 | 0.603 |
| 10 | 555 | 0.053 | 0.105 | 0.159 | 0.266 | 0.531 |
| 11 | 595 | 0.026 | 0.051 | 0.078 | 0.133 | 0.261 |
| 12 | 598 | 0.027 | 0.052 | 0.077 | 0.134 | 0.267 |

Reference Example 2 [Determination of PHI activity]

(Composition of reagent)

Reagent solution 1: pH 7.5

| | |
|---|---|
| Monosodium phosphate | 0.1 M |
| Triton X-100 | 0.1% |
| Magnesium chloride | 5 mg/ml |
| Peroxidase | 5 IU/ml |
| LDH | 5 IU/ml |
| Glucose-6-phosphate dehydrogenase | 3 IU/ml |
| LOX | 2.5 IU/ml |
| Pyruvic acid | 0.25 mg/ml |
| NAD | 0.8 mg/ml |
| Compound No. 59 | 1 mg/ml |

Reagent solution 2: pH 7.5

| | |
|---|---|
| Monosodium phosphate | 0.1 M |
| Triton X-100 | 0.1% |
| 4-Aminoantipyrine | 1.5 mg/ml |
| Fructose-6-phosphate | 9 mg/ml |

(Procedure)

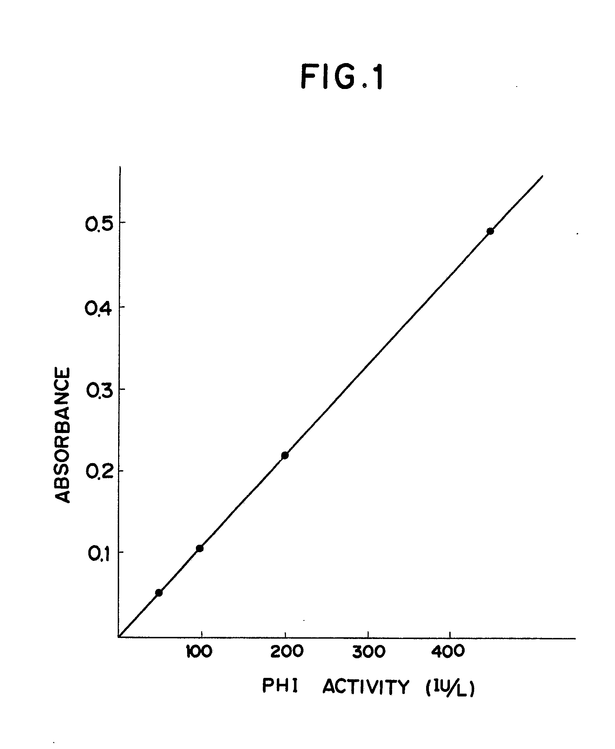

Reagent 1, 2.25 ml, was kept at 37°C and 50 µl each of (1) 50 IU/l, (2) 100 IU/l, (3) 200 IU/l and (4) 400 IU/l of PHI (product of Sigma Inc.) was added thereto followed by agitation. Five minutes after, 0.75 ml of Reagent 2 was added to the mixture to render it homogeneous. Absorbance at 595 nm was then measured 10 minutes after. The obtained calibration curve is shown in Fig. 1 of the accompanying drawings.

Example 1 (Determination of total bile acid in serum)

Reagent 1: pH 6.25

| | |
|---|---|
| Bis(2-hydroxyethyl)iminotris-(hydroxymethyl)methane | 0.1 M |
| Triton X-100 | 0.5% |
| Pyruvic acid | 1 mg/ml |
| Albumin | 1 mg/ml |
| Phenol | 0.1 mg/ml |
| Peroxidase | 10 IU/ml |
| LDH | 8 IU/ml |
| LOX | 5 IU/ml |
| NAD | 4 mg/ml |

Reagent 2: pH 7.0

| | |
|---|---|
| N-2-hydroxyethylpiperazine-N'-2-ethanesulfonic acid | 0.05 M |
| Triton X-100 | 1.0% |
| 3α-Hydroxysteroid dehydrogenase | 6 IU/ml |

In Reagent 2 were dissolved A: 0.1 mg/ml of Compound No. 1, B: 0.1 mg/ml of Compound No. 20, C: 0.1 mg/ml of Compound No. 21, D: 0.1 mg/ml of Compound No. 26 and E: 0.1 mg/ml of Compound No. 27, respectively, to make reagent solutions 2-A, 2-B, 2-C, 2-D and 2-E, respectively.

Reagent 1, 2.25 ml, was kept at 37° C and 50 μl of fresh serum was added thereto followed by agitation. Five minutes after, 0.75 ml each of Reagent 2-A, B, C, D and E was added to the mixture to render it homogeneous. Then, the mixture was allowed to stand at 37° C for further 5 minutes. Absorbance of the reaction solution was then measured at λmax of the chromogen in contrast with a reagent blank test. From the calibration curve previously prepared on known concentrations, the concentration of total bile acid in serum is calculated. The results are shown in Table 6, in comparison with the measurement using high performance liquid chromatography (HPLC).

Table 6

Concentration of total bile acid (μmol/l)

| Serum sample No. | HPLC | A | B | C | D | E |
|---|---|---|---|---|---|---|
| 1 | 4.3 | 4.5 | 4.3 | 4.2 | 4.4 | 4.5 |
| 2 | 18.2 | 18.0 | 18.4 | 18.1 | 18.0 | 18.2 |
| 3 | 5.6 | 5.8 | 5.8 | 5.7 | 5.4 | 5.4 |
| 4 | 31.4 | 30.9 | 31.1 | 31.8 | 30.9 | 31.5 |
| 5 | 9.0 | 9.0 | 9.0 | 9.3 | 9.1 | 9.0 |

In serum Sample Nos. 1 through 5, 9.8, 12.3, 5.6, 14.1 and 8.2 mg/dl of lactic acid were contained, respectively but no influence was noted.

Example 2 (Determination of PHI activity in serum)

The same Reagents 1 and 2 as in Reference Example 2 were used. Reagent 1, 2.25 ml was kept at 37° C and 50 μl of fresh serum was added thereto followed by agitation. Five minutes after, 0.75 ml of Reagent 2 was added to the mixture to render it homogeneous. Ten minutes after, absorbance of the reaction solution was measured at λmax of the chromogen, 595 nm in contrast with a reagent blank test, and PHI activity in the serum was calculated by a calibration curve prepared in advance.

Results are shown in Table 7 in comparison with the measurement of absorption of NADH in the ultraviolet region (UV Method)

In Serum Samples Nos. 1 through 5, the amounts of lactic acid, indicated in Table 7 were contained, but no influence was noted.

Table 7

PHI Activity (mIU/ℓ)

| Serum sample No. | UV method | The present invention | lactic acid (mg/dℓ) |
|---|---|---|---|
| 1 | 15.8 | 15.6 | 7.2 |
| 2 | 19.4 | 19.5 | 23.1 |
| 3 | 72.1 | 71.9 | 6.5 |
| 4 | 33.2 | 33.4 | 10.3 |
| 5 | 18.8 | 19.0 | 8.6 |

**Claims**

1. A method for the determination of NAD(P)H in solution which comprises carrying out an electron transfer reaction in said solution in the presence of said NAD(P)H, such reaction resulting at least indirectly in the quantitative production of $H_2O_2$ in solution proportional to the NAD(P)H concentration, and colorimetrically determining the $H_2O_2$ produced, by the reaction thereof with peroxidase in the presence of a chromogen unsusceptible to NAD(P)H, characterised in that the electron transfer reaction comprises reacting pyruvic acid or a salt thereof with lactate dehydrogenase in said solution in the presence of said NAD(P)H, thereby to produce lactic acid, and thereafter reacting the lactic acid produced with lactate oxidase, thereby to produce said $H_2O_2$.

2. A method according ot claim 1, as applied to the determination of NAD(P)H in solution in the presence of lactic acid, and wherein the lactic acid initially present in the solution is initially reacted with lactate oxidase thereby to convert the lactic acid initially present into pyruvic acid and hydrogen peroxide, and thereafter decomposing the product hydrogen peroxide in the presence of an $H_2O_2$-decomposer, the pyruvic acid produced by that initial reaction providing at least a proportion of the pyruvic acid subsequently reacted with said lactate dehydrogenase to form the lactic acid prior to the reaction thereof with said lactate oxidase and the consequent formation of said $H_2O_2$.

3. A method according to claim 2, wherein the intitial lactic acid content of the soluiton is sufficient to provide all the pyruvic acid needed for the subsequent reaction with lactate dehydrogenase in the presence of the NAD(P)H to be determined.

4. A method according to claim 2, wherein following the initial reaction to convert lactic acid to pyruvic acid, additional pyruvic acid or a salt thereof is added to the reaction mixture prior to the reaction thereof with said lactate dehydrogenase.

5. A method according to claim 2, 3 or 4, wherein the $H_2O_2$ decomposer is catalase.

6. A method according to claim 2, 3, 4 or 5, wherein following the decomposition of the $H_2O_2$ produced by the initial reaction between the lactate oxidase by the $H_2O_2$-decomposer, any excess $H_2O_2$ decomposer is deactivated.

7. A method according to claim 6, wherein the $H_2O_2$-decomposer is catalase and the deactivator is sodium azide.

8. A method according to any one of the preceding claims, wherein the chromogen used is a compound as listed in Table 1 or 2.

9. A method according to any one of claims 1 to 8, wherein the chromogen used is a coupling agent constituted by 4-aminoantipyrine and a compound as listed in Table 3.

10. A method according to claim 9, as dependent on any one of claims 2 to 5, wherein one component of the coupling agent also acts as said $H_2O_2$ decomposer.

11. A method according to any one of claims 1 to 10, wherein 0.1 to 1000 IU/ml lactate dehydrogenase, 0.1 to 1000 IU/ml lactate oxidase, 0.1 to 100 mM/l pyruvic acid or a salt thereof, 0.1 to 1000 IU/ml peroxidase and 10 to 1000 molar equivalents of the chromogen are used, based on NAD(P)H to be determined.

12. A method according to any one of claims 1 to 11, wherein NAD(P)H is adjusted with a reagent solution or distilled water to a concentration of 0.00001 to 1 mg/ml.

13. A method according to any one of the preceding claims, which is carried out in a buffer having a pH of 2 to 11 at 5 to 50° C.

14. A method according to claim 13, which is carried out in an acidic buffer.

15. A method according to any one of the preceding claims, which is carried out in the presence of surfactant and/or phenol.

16. A method according to any one of the preceding claims, as applied to the determination of NAD(P)H

stoichiometrically produced by an enzyme reaction involving an enzyme, a substrate and NAD(P) which is converted to NAD(P)H as a result of the enzyme reaction.

17. A method according to claim 16, which is carried out in the presence of an activator and/or stabilizer for the enzyme involved in the NAD(P)H producing reaction.

18. A method according to claim 16 or 17, wherein the enzyme reaction involves as the substrate glucose, galactose, alcohols, malic acid, aldehyde, xanthine, cholesterol, bile acid, neutral lipid or glycerol.

19. A method according to claim 16 or 17, wherein the enzyme reaction involves phosphohexose isomerase as the analyte to be determined.

20. A method according to any one of the preceding claims, as applied to the determination of NAD(P)H in a clinical sample.

21. A method for the determination of bile acid or phosphohexose isomerase activity in a clinical sample which comprises:

(a) oxidising lactic acid in the sample in the presence of lactate oxidase into pyruvic acid to form hydrogen peroxide;

(b) decomposing the hydrogen peroxide;

(c) subjecting the bile acid or phosphohexose isomerase, as the case may be, to an enzyme reaction with the appropriate enzyme or substrate, respectively, in the presence of NAD(P) stoichiometrically to form NAD(P)H;

(d) reducing pyruvic acid or a salt thereof with the NAD(P)H so formed in the presence of lactate dehydrogenase to form lactic acid;

(e) oxidising the lactic acid in the presence of lactate oxidase to form hydrogen peroxide; and

(f) colorimetrically determining the hydrogen peroxide by reaction with peroxidase in the presence of a chromogen unsusceptible to NAD(P)H.

22. A method according to claim 20 or 21, wherein the clinical sample is a serum sample.

23. A reagent kit for the determination of NAD(P)H in solution comprising as a first reagent: lactate oxidase, an $H_2O_2$ decomposer, and one or other of peroxidase and a chromogen unsusceptible to NAD(P)H, and as a second reagent: the other of said peroxidase and chromogen, whichever is not included in the first component, the first or second reagents or both containing one or other or both of lactic acid dehydrogenase and pyruvic acid or a salt thereof, with the proviso that the two reagents between them contain both said lactic acid dehydrogenase and said pyruvic acid or salt.

24. A reagent kit according to claim 23, wherein the second reagent further comprises NAD(P) and an enzyme or enzyme substrate capable of reacting respectively with an enzyme substrate or enzyme in a sample, the quantity or activity of which is to be determined indirectly by the reaction of the enzyme and its substrate in the presence of said NAD(P), thereby to produce NAD(P)H in solution.

FIG.1